# EUROPÄISCHE PATENTANMELDUNG

(11) **EP 3 159 327 A1**
(43) Veröffentlichungstag der Anmeldung: **26.04.2017**
(21) Anmeldenummer: 15191040.3
(22) Anmeldetag: 22.10.2015
(51) Int. Cl.: C07C 2/84, B01J 12/00, C10G 9/00

(54) **PROZESS UND ANLAGE ZUR HERSTELLUNG VON OLEFINEN**

(71) Anmelder: Linde Aktiengesellschaft, 80331 München (DE)
(72) Erfinder: Zimmermann, Heinz, 81476 München (DE)
(74) Vertreter: m patent group

(57) **Zusammenfassung**

Es wird ein Prozess (100) zur Herstellung von Olefinen vorgeschlagen, bei dem Methan zusammen mit Sauerstoff parallel einem ersten und einem zweiten katalytischthermischen Umsetzungsverfahren (10A, 10B) zugeführt wird, wobei das erste und das zweite katalytisch-thermische Umsetzungsverfahren (10A, 10B) jeweils einen katalytischen Schritt (11A, 11B) und einen sich dem katalytischen Schritt anschließenden thermischen Schritt (12A, 12B) umfassen, wobei das Methan in den katalytischen Schritten (11A, 11B) jeweils zu einem Teil unter Bildung von höheren Kohlenwasserstoffen umgesetzt wird, wodurch jeweils ein erstes Gasgemisch gebildet wird, das die höheren Kohlenwasserstoffe umfasst, das erste Gasgemisch jeweils in den thermischen Schritten (12A, 12B) vollständig oder teilweise einem absteigenden Temperaturgradienten unterworfen werden, wodurch die höheren Kohlenwasserstoffe jeweils zu einem Teil weiter umgesetzt werden und jeweils ein zweites Gasgemisch gebildet wird, das Ethan, Ethylen und Paraffine und Olefine mit drei und vier Kohlenstoffatomen umfasst, aus den zweiten Gasgemischen eine erste Fraktion, die überwiegend oder ausschließlich Ethan enthält, und wenigstens eine zweite Fraktion, die Paraffine mit drei und/oder vier Kohlenstoffatomen und/oder Olefine mit vier Kohlenstoffatomen enthält, gebildet werden, in dem thermischen Schritt (12A) des ersten katalytisch-thermischen Umsetzungsverfahrens (10A) eine höhere Spaltschärfe festgelegt wird als in dem thermischen Schritt (12B) des zweiten katalytischthermischen Umsetzungsverfahrens (10B), und die erste Fraktion in den thermischen Schritt (12A) des ersten katalytisch-thermischen Umsetzungsverfahrens (10A) und die wenigstens eine zweite Fraktion in dem thermischen Schritt (12B) des zweiten katalytisch-thermischen Umsetzungsverfahrens (10B) zurückgeführt wird. Eine entsprechende Anlage ist ebenfalls Gegenstand der Erfindung.

## Beschreibung

Die Erfindung betrifft einen Prozess und eine Anlage zur Herstellung von Olefinen, bei dem Methan zusammen mit Sauerstoff parallel einem ersten und einem zweiten katalytisch-thermischen Umsetzungsverfahren zugeführt wird.

### Stand der Technik

Verfahren zur Herstellung höherer Kohlenwasserstoffe aus Methan durch oxidative Methankopplung (engl. Oxidative Coupling of Methane, OCM) befinden sich derzeit in intensiver Entwicklung. Bei der oxidativen Methankopplung werden ein methanreicher und ein sauerstoffreicher Strom in einen Reaktor eingespeist, wo der Sauerstoff des sauerstoffreichen Stroms und ein Teil des Methans des methanreichen Stroms unter Bildung von Wasser und Nebenprodukten zu höheren Kohlenwasserstoffen, insbesondere dem typischen Zielprodukt Ethylen, reagieren.

Beispielsweise in der US 2015/0152025 A1 und der US 2015/0210610 A1 sind kombinierte katalytisch-thermische Umsetzungsverfahren bekannt, die die oxidative Methankopplung als katalytischen Schritt umfassen. Dem katalytischen Schritt schließt sich ein thermischer Schritt, das sogenannte "Post Bed Cracking" (nachfolgend auch als postkatalytisches Dampfspalten bezeichnet) an. Bei diesem postkatalytischen Dampfspalten durchläuft das in dem katalytischen Schritt gebildete Gasgemisch einen absteigenden Temperaturgradienten, so dass geeignete, in dem Gasgemisch enthaltene Komponenten unter Bedingungen umgesetzt werden, die jenen eines normalen Dampfspaltverfahrens ähneln. Insbesondere enthält das dem postkatalytischen Dampfspalten unterworfene Gasgemisch, wie erwähnt, Wasser.

Zu den beim Dampfspalten von Kohlenwasserstoffen verwendeten Bedingungen sei auf einschlägige Fachliteratur, beispielsweise den Artikel "Ethylene" in Ullmann's Encyclopedia of Industrial Chemistry, Onlineausgabe, 15. April 2007, DOI 10.1002/14356007.a10_045.pub2, verwiesen.

Dem postkatalytischen Dampfspalten können neben dem in dem katalytischen Schritt gebildeten Gasgemisch auch weitere Kohlenwasserstoffe zugeführt werden. In der erwähnten US 2015/0152025 A1 wird beispielsweise vorgeschlagen, dem postkatalytischen Dampfspalten Alkane mit zwei und mehr Kohlenstoffatomen zuzuführen. Diese können von anlagenextern zugeführt werden und beispielsweise die sogenannten "Natural Gas Liquids" (nachfolgend auch als Erdgaskondensate bezeichnet) umfassen. Auch in der US 2015/0210610 A1 ist offenbart, dem postkatalytischen Dampfspalten weitere Kohlenwasserstoffe zuzuführen. Hierbei wird Erdgas einer Extraktionseinheit zugeführt, die Kohlenwasserstoffe mit zwei und mehr Kohlenstoffatomen von leichteren Komponenten wie Methan trennt. Das Methan wird dem katalytischen Schritt zugeführt. Die Kohlenwasserstoffe mit zwei und mehr Kohlenstoffatomen werden in unterschiedliche Fraktionen aufgetrennt, die teilweise dem postkatalytischen Dampfspalten zugeführt werden.

Aus den genannten Druckschriften ist auch die Rückführung von Fraktionen zum postkatalytischen Dampfspalten bekannt, die aus einem Gasgemisch gewonnen werden, das stromab des postkatalytischen Dampfspaltens vorliegt. Hierbei handelt es sich um Methan und Paraffine mit zwei Kohlenstoffatomen.

In den erläuterten Verfahren werden jedoch nicht immer die gewünschten Produkte gebildet. Die vorliegende Erfindung stellt sich daher die Aufgabe, entsprechende Verfahren zu verbessern und die eingesetzten Verbindungen besser zu nutzen.

### Offenbarung der Erfindung

Diese Aufgabe wird durch einen Prozess und eine Anlage zur Herstellung von Olefinen mit den Merkmalen der jeweiligen unabhängigen Patentansprüche gelöst. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Gemische können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem das flüssige oder gasförmige Gemisch erhalten wurde. Das flüssige oder gasförmige Strom ist "angereichert", wenn dieses zumindest den 1,1-fachen, 1,5-fachen, 2-fachen, 5-fachen, 10-fachen, 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Das "katalytisch-thermische Umsetzungsverfahren" der vorliegenden Erfindung umfasst einen katalytischen Schritt in Form einer oxidativen Methankopplung und einen sich dem katalytischen Schritt anschließenden thermischen Schritt in Form der eingangs erläuterten postkatalytischen Dampfspaltens. Beide Schritte können jeweils in einem oder mehreren Reaktoren bzw. Reaktoranordnungen durchgeführt werden, die parallel oder seriell angeordnet sein können. Immer schließt sich jedoch das postkatalytische Dampfspalten direkt dem katalytischen Schritt an. Insbesondere können der katalytische Schritt und das postkatalytische Dampfspalten in einem gemeinsamen Reaktor mit einer katalytischen Zone (mit einem Katalysatorbett) und einer postkatalytischen Zone (ohne Katalysatorbett) durchgeführt werden. Das postkatalytische Dampfspalten wird insbesondere unter Verwendung von Abwärme des katalytischen Schritts durchgeführt.

Wie erwähnt wird ein in dem katalytischen Schritt gebildetes Gasgemisch beim postkatalytischen Dampfspalten einem absteigenden Temperaturgradienten unterworfen. Dieser umfasst beispielsweise einen Temperaturbereich von 1.000 bis 700 °C, d.h. zu Beginn des postkatalytischen Dampfspaltens beträgt die Temperatur höchstens 1.000 °C, insbesondere 800 bis 1.000 °C, beispielsweise 880 bis 950 °C. Am Ende des postkatalytischen Dampfspaltens, d.h. am Austritt des verwendeten Reaktors, beträgt die Temperatur mindestens 700 °C, insbesondere 700 bis 900 °C, beispielsweise 750 bis 880 °C. In herkömmlichen Verfahren zum Dampfspalten können die verwendeten Spaltbedingungen unter anderem durch den Partialdruck der eingesetzten Kohlenwasserstoffe, der durch die Zugabe unterschiedlicher Dampfmengen und den verwendeten Druck eingestellt werden kann, die Verweildauer im Spaltofen sowie die in diesem verwendeten Temperaturen und Temperaturprofile beeinflusst werden. Auch die Geometrie und Gestaltung der verwendeten Reaktoren spielt eine Rolle.

Auch beim postkatalytischen Dampfspalten können diese Parameter, sofern sie nicht durch den katalytischen Schritt bzw. die dort geforderten Bedingungen festgelegt sind, beeinflusst werden. Ein bedeutender Einflussfaktor auf die Spaltbedingungen für zusätzlich eingespeiste Kohlenwasserstoffe ist hier jedoch die Einspeisestelle in den Reaktor. Diese legt fest, bei welcher Temperatur des erwähnten absteigenden Temperaturgradienten die Zuspeisung erfolgt, und welchen Temperaturen die eingespeisten Kohlenwasserstoffe wie lange ausgesetzt werden. Das aus dem katalytischen Schritt stammende Gasgemisch durchläuft den Temperaturgradienten vollständig, wobei es einen entsprechenden Reaktor oder eine entsprechende Reaktorzone innerhalb einer definierten Zeit, die die Verweildauer bestimmt, durchströmt. Werden zusätzliche Kohlenwasserstoffe beispielsweise nach der Hälfte der Wegstrecke, die durch das Gasgemisch aus dem katalytischen Schritt durchlaufen wird, zugespeist, beträgt die Verweildauer die Hälfte und die Temperatur ist entsprechend dem Temperaturprofil des Temperaturgradienten reduziert. Nachfolgend ist davon die Rede, dass beim postkatalytischen Dampfspalten einer zugespeisten Komponente eine definierte Umsetzung erfolgt. Diese auf den ersten Blick als bloße Aufgabenstellung erscheinende Angabe stellt sich bei angemessener Betrachtungsweise jedoch als konkrete Anweisung an den Fachmann dar, der die Temperaturen, Verweildauern, die spezifische Geometrie und Gestaltung der verwendeten Reaktoren usw. kennt.

Wie oben erwähnt, beeinflussen eine Reihe von Parametern die Spaltbedingungen. Diese sind in jedem Reakor unterschiedlich und unterscheiden sich je nach Verfahrensführung. Es ist daher nicht möglich, beispielsweise konkrete Angaben zu machen, beispielsweise welcher Temperatur oder Verweildauer ein entsprechender, zusätzlich eingespeister Kohlenwasserstoff beim postkatalytischen Dampfspalten genau ausgesetzt werden soll, da konkrete Werte immer auch von anderen verwendeten Parametern abhängen und keine absolute Gültigkeit besitzen. Die Verwendung einer konkreten Temperatur könnte, bei entsprechender Auslegung anderer Parameter, auch dazu führen, dass sich die im Rahmen der vorliegenden Erfindung erwünschten Effekte nicht einstellen.

Die Charakterisierung der Spaltbedingungen über die Bildung oder Umsetzung einzelner Komponenten ist beim Dampfspalten völlig üblich und beispielsweise in dem eingangs erwähnten Artikel "Ethylene" in Ullmann's Encyclopedia of Industrial Chemistry beschrieben. Unter anderem hat sich Begriff der "Spaltschärfe" (engl. Cracking Severity) durchgesetzt. Für flüssige Ofeneinsätze kann die Spaltschärfe über das Verhältnis von Propylen zu Ethylen (P/E) oder als das Verhältnis von Methan zu Propylen (M/P) im Spaltgas auf Gewichtsbasis (kg/kg) beschrieben werden.

Für gasförmige Ofeneinsätze kann dagegen die Umsetzung bzw. Konversion einer jeweils betrachteten Komponente des Ofeneinsatzes als Maß der Spaltschärfe angegeben werden. Insbesondere für Kohlenwasserstoffe mit vier Kohlenstoffatomen ist eine Beschreibung der Spaltschärfe bzw. der Spaltbedingungen über die Umsetzung von Schlüsselkomponenten gut geeignet. Der Fachmann auf dem Gebiet des Dampfspaltens kennt die die Spaltbedingungen beeinflussenden Parameter und wählt bzw. verändert diese in zweckmäßiger Weise. Im Zweifelsfall führen einfache Routinemessungen ohne unzumutbaren Aufwand zum gewünschten Ergebnis.

### Vorteile der Erfindung

Die vorliegende Erfindung geht von der Erkenntnis aus, dass, wenn die oxidative Kopplung von Methan mit einer nachgeschalteten postkatalytischen Dampfspaltung in mehreren, parallel betriebenen Reaktoren durchgeführt wird, sich dann besondere Vorteile ergeben, wenn die postkatalytische Dampfspaltung unter unterschiedlichen Bedingungen durchgeführt wird. Auf diese Weise können gezielt bestimmte Kohlenwasserstoffe bzw. Kohlenwasserstofffraktionen in bestimmte Reaktoren zurückgeführt und unter unterschiedlichen, jeweils an die Kohlenwasserstoffe angepassten Bedingungen bearbeitet werden.

Hierzu schlägt die vorliegende Erfindung einen Prozess zur Herstellung von Olefinen vor, bei dem Methan zusammen mit Sauerstoff parallel einem ersten und einem zweiten katalytisch-thermischen Umsetzungsverfahren zugeführt wird. Die katalytischthermischen Umsetzungsverfahren umfassen, wie bereits erläutert, jeweils einen katalytischen Schritt zur oxidativen Kopplung von Methan und einen katalytischen Schritt nachgeschalteten, postkatalytischen Dampfspaltschritt. Das erste und das zweite katalytisch-thermische Umsetzungsverfahren umfassen also jeweils einen katalytischen Schritt und einen sich dem katalytischen Schritt anschließenden thermischen Schritt. Das Methan wird dabei in katalytischen Schritten jeweils zu einem Teil unter Bildung von höheren Kohlenwasserstoffen umgesetzt, wodurch jeweils ein erstes Gasgemisch gebildet wird, das die höheren Kohlenwasserstoffe, die aus dem Methan durch die katalytische Umsetzung gebildet wird, umfasst. Das erste Gasgemisch umfasst dabei jeweils neben den gebildeten Kohlenwasserstoffen auch nicht umgesetztes Methan sowie übliche Nebenprodukte wie beispielsweise Wasser, Kohlenmonoxid und Kohlendioxid.

Diese ersten Gasgemisch werden jeweils in den thermischen Schritten vollständig oder teilweise einen absteigenden Temperaturgradienten unterworfen, wodurch die höheren Kohlenwasserstoffe jeweils zu einem Teil weiter umgesetzt werden und jeweils ein zweites Gasgemisch gebildet wird, das Ethan, Ethylen und Paraffine und Olefine mit drei und vier Kohlenstoffatomen umfasst. Neben den genannten Kohlenwasserstoffen können in den parallelen thermischen Schritten insbesondere weitere Kohlenwasserstoffe gebildet werden, beispielsweise Diolefine, Acetylen und Kohlenwasserstoffe mit mehr als vier Kohlenstoffatomen. Die Behandlung entsprechender Verbindungen wird teilweise nachfolgend erläutert.

Aus den zweiten Gasgemischen wird anschließend, beispielsweise nach Vereinigung und in einer gemeinsamen Trenneinrichtung, eine erste Fraktion gebildet, die überwiegend oder ausschließlich Ethan enthält, und wenigstens eine zweite Fraktion, insbesondere überwiegend oder ausschließlich Paraffine mit drei und/oder vier Kohlenstoffatomen und/oder Olefine mit vier Kohlenstoffatomen enthält. Diese Fraktionen werden nun erfindungsgemäß unterschiedlich behandelt. Diese unterschiedliche Behandlung ist möglich, weil in dem thermischen Schritt des ersten katalytisch-thermischen Umsetzungsverfahrens eine höhere Spaltschärfe festgelegt wird als in dem thermischen Schritt des zweiten katalytisch-thermischen Umsetzungsverfahrens. Zum Begriff der "Spaltschärfe" (engl. Cracking Severity) und das fachübliche Verständnis sei auf die obigen Erläuterungen sowie die zitierte einschlägige Fachliteratur verwiesen.

Im Rahmen der vorliegenden Erfindung ist vorgesehen, die erste Fraktion in den thermischen Schritt des ersten katalytisch-thermischen Umsetzungsverfahrens und die wenigstens eine zweite Fraktion in den thermischen Schritt des zweiten katalytischthermischen Umsetzungsverfahrens zurückzuführen. Auf diese Weise kann die erste Fraktion, die überwiegend oder ausschließlich Ethan enthält, schärfer gespalten werden als die wenigstens eine zweite Fraktion, die Kohlenwasserstoffe mit einer höheren Kohlenstoffanzahl enthält. Die genannten Fraktionen können daher im Rahmen des erfindungsgemäßen Verfahrens jeweils gezielt angepasst an die Erfordernisse bzw. die gewünschten Produkte eines entsprechenden Prozesses bearbeitet werden. Hierdurch erhöht sich die Flexibilität gegenüber herkömmlichen Verfahren, in denen ein postkatalytischer Dampfspaltschritt einheitlich für sämtliche Kohlenwasserstoffe durchgeführt wird.

Beispielsweise kann durch die schärfere Spaltung des Ethans in der postkatalytischen Dampfspaltung die Ausbeute an Ethylen im Rahmen des erfindungsgemäßen Verfahrens verbessert werden. Gleichzeitig kann durch die mildere Spaltung höherer Kohlenwasserstoffe, insbesondere der Kohlenwasserstoffe mit vier Kohlenstoffatomen, die Ausbeute an weiteren Wertprodukten, insbesondere an Butadien, erhöht werden. Eine übermäßig scharfe Spaltung von Kohlenwasserstoffen mit vier Kohlenstoffatomen würde hingegen zur Bildung von einer größeren Menge an Ethylen bzw. Methan führen, die gegenüber Butadien bzw. höheren Kohlenwasserstoffen von geringerem wirtschaftlichem Wert sein können.

Mit besonderem Vorteil wird die Spaltschärfte in dem thermischen Schritt des ersten katalytisch-thermischen Umsetzungsverfahrens derart festgelegt, dass Ethan mindestens zu 20 Mol-%, insbesondere mindestens zu 30 oder 40 Mol-% und höchstens zu 60 Mol-%, insbesondere höchstens zu 50 Mol-%, umgesetzt wird. Die Umsetzung erfolgt dabei insbesondere zu Ethylen. Entsprechend wird die Spaltschärfe in dem thermischen Schritt des zweiten katalytisch-thermischen Umsetzungsverfahrens vorteilhafterweise so festgelegt, dass Propan mindestens zu 30 Mol-%, insbesondere mindestens zu 40 oder 50 Mol.-%, und höchstens zu 80 Mol-%, insbesondere höchstens zu 70 oder 60 Mol-%, umgesetzt wird. Die Umsetzung erfolgt dabei insbesondere zu Propylen. Wie bereits eingangs erläutert, eignet sich die Umsetzung von gasförmigen Komponenten, wie vorliegend des Ethans und Propans, zur Definition einer Spaltschärfe. Der Fachmann kann die jeweiligen Bedingungen in den verwendeten Reaktoren bzw. Reaktorzonen, beispielsweise durch Verändern einer Temperatur, bei der das erste Gasgemisch jeweils den katalytischen Schritt verlässt, oder auch beispielsweise durch zusätzliche Dampfeinspeisung oder (Partial-)Druckbeeinflussung, anpassen. Weil, wie erwähnt, eine Reihe unterschiedlicher Faktoren einander wechselseitig beeinflussen, ist hier eine Beschreibung der Spaltschärfen über die Umsetzung der Schlüsselkomponente Ethan zweckmäßig.

Vorteilhafterweise wird das in dem katalytischen Schritt des ersten katalytisch-thermischen Umsetzungsverfahrens gebildete erste Gasgemisch dem thermischen Schritt des ersten katalytisch-thermischen Umsetzungsverfahrens auf einem Temperaturniveau von 880 °C bis 950 °C zugeführt. Diese Temperatur lässt sich beispielsweise durch Ändern der Reaktionsbedingungen in dem katalytischen Schritt, durch Ändern der Einspeisetemperatur in dem katalytischen Schritt oder durch eine Zwischenabfuhr von Wärme zwischen dem katalytischen Schritt und dem thermischen Schritt beeinflussen. Entsprechendes gilt auch für die Temperatur, bei dem das in dem katalytischen Schritt des zweiten katalytisch-thermischen Umsetzungsverfahrens gebildete erste Gasgemisch dem thermischen Schritt des zweiten katalytischthermischen Umsetzungsverfahrens zugeführt wird. Dieses Temperaturniveau liegt vorteilhafterweise bei 850 bis 950 °C. Durch die genannten Temperaturniveaus lassen sich die zuvor erläuterten Spaltschärfenunterschiede und damit die Vorteile der vorliegenden Erfindung erzielen.

Im Rahmen der vorliegenden Erfindung ist es besonders vorteilhaft, wenn die zweite Fraktion Propan, Butan und Butene, insbesondere lineare Butene umfasst. Die Paraffine können dabei zu Olefinen umgesetzt werden, welche sich als Wertprodukte vermarkten lassen; insbesondere lineare Butene eignen sich zur Gewinnung von Butadien. Insbesondere trifft dies auf 2-Buten zu. Enthalten die zweite Fraktion oder die zweiten Gasgemische daher 1-Buten, kann vorgesehen sein, dieses 1-Buten zu 2-Buten zu isomerisieren. Eine derartige Isomerisierung kann jedoch auch unter milden Spaltbedingungen, wie sie in dem thermischen Schritt des zweiten katalytischthermischen Umsetzungsverfahrens vorliegen, erfolgen. Isobuten kann beispielsweise abgetrennt und/oder umgesetzt werden, wie nachfolgend noch erläutert.

Weil die zweiten Gasgemische typischerweise Kohlenmonoxid und Kohlendioxid enthalten, werden diese gemäß einer besonders bevorzugten Ausführungsform der Erfindung vor einer Bildung der genannten Fraktionen in zweckmäßiger Weise weiterbehandelt. So können das Kohlenmonoxid und/oder das Kohlendioxid aus dem zweiten Gasgemisch abgetrennt, einer bekannten Methanisierung unterworfen, und zumindest einem der katalytisch-thermischen Umsetzungsverfahren wieder zugeführt werden. Auf diese Weise steigert sich die Gesamtkohlenstoffausbeute des erfindungsgemäßen Prozesses, dass der hierdurch insgesamt effizienter wird.

Wie bereits erläutert, eignet sich der erfindungsgemäße Prozess in besonderer Weise zur Herstellung von Olefinen, d.h. für Fälle, in denen aus den zweiten Gasgemischen eine ethylenreiche, eine propylenreiche und/oder eine butadienreiche Fraktion abgetrennt werden. Die Ausbeute an derartigen Verbindungen ist in dem erfindungsgemäßen Prozess besonders hoch, weil eine Adaption der Spaltbedingungen an jeweils noch nicht umgesetzte Verbindungen erfolgen kann, die auf diese Weise vollständig in die genannten Produkte umgewandelt werden können.

Zusätzlich können die zweiten Gasgemische Kohlenwasserstoffe mit fünf oder fünf und mehr Kohlenstoffatomen enthalten. Eine Ausführungsform der Erfindung umfasst, aus den zweiten Gasgemischen eine an solchen Kohlenwasserstoffen reiche Fraktion abzutrennen und als Produkt aus der Anlage auszuleiten oder weiter aufzubereiten. Besonders vorteilhaft ist, wenn aus derartigen Kohlenwasserstoffen beispielsweise aromatische Verbindungen gewonnen und als Wertprodukte bereitgestellt werden. Derartige aromatische Verbindungen können sich insbesondere deshalb bilden, weil in dem thermischen Schritt des zweiten katalytisch-thermischen Umsetzungsverfahrens besonders milde Spaltbedingungen verwendet werden, bei welchen sich derartige Wertprodukte bevorzugt bilden.

Vorteilhafterweise wird das dem katalytisch-thermischen Umsetzungsverfahren zugeführte Methan zumindest zum Teil in einem Abtrennverfahren aus Erdgas abgetrennt, wobei neben dem Methan eine oder mehrere weitere Fraktionen gebildet werden. Vorteilhafterweise wird dabei eine Fraktion gebildet, die Kohlenwasserstoffe mit zwei oder zwei und mehr Kohlenstoffatomen enthält. Diese Fraktion wird vorteilhafterweise teilweise oder vollständig dem thermischen Schritt zumindest eines der katalytisch-thermischen Umsetzungsverfahren zugeführt. Aus entsprechend abgetrennten Kohlenwasserstoffen können auch mehrere Fraktionen gebildet werden, die gezielt einem der thermischen Schritte, je nach geeigneter Spaltschärfe, zugeführt werden können. Beispielsweise kann abgetrenntes Ethan bevorzugt dem thermischen Schritt des ersten katalytisch-thermischen Umsetzungsverfahrens zugeführt werden, wohingegen höhere Kohlenwasserstoffe bevorzugt dem thermischen Schritt des zweiten katalytisch-thermischen Verfahrens zugeführt werden können.

Die vorliegende Erfindung erstreckt sich ferner auf eine Anlage zur Durchführung des Prozesses zur Herstellung von Olefinen, der dafür eingerichtet ist, Methan zusammen mit Sauerstoff parallel einem ersten und einem zweiten katalytisch-thermischen Umsetzungsverfahren zuzuführen, wobei das erste und das zweite katalytischthermische Umsetzungsverfahren jeweils einen katalytischen Schritt und einen sich dem katalytischen Schritt anschließenden thermischen Schritt umfassen. Die Anlage ist dafür eingerichtet, dass Methan in den katalytischen Schritten jeweils zu einem Teil unter Bildung von höheren Kohlenwasserstoffen umzusetzen, wodurch jeweils ein erstes Gasgemisch gebildet wird, dass die höheren Kohlenwasserstoffe umfasst. Die ersten Gasgemische werden jeweils in den thermischen Schritten vollständig oder teilweise einem absteigendem Temperaturgradienten unterworfen, wodurch die höheren Kohlenwasserstoffe jeweils zu einem Teil weiter umgesetzt werden und jeweils ein zweites Gasgemisch gebildet wird, das Ethan, Ethylen und Paraffine und Olefine mit drei und vier Kohlenstoffatomen umfasst.

Die Anlage ist ferner dazu eingerichtet, aus den zweiten Gasgemischen eine erste Fraktion, die überwiegend oder ausschließlich Ethan enthält, und wenigstens eine zweite Fraktion, die Paraffine mit drei und/oder vier Kohlenstoffatomen und/oder Olefine mit vier Kohlenstoffatomen enthält, zu bilden. Ferner ist die Anlage dazu eingerichtet, in dem thermischen Schritt des ersten katalytisch-thermischen Umsetzungsverfahrens eine höhere Spaltschärfe festzulegen als in dem thermischen Schritt des zweiten katalytisch-thermischen Umsetzungsverfahrens. Schließlich ist die Anlage dazu eingerichtet, die erste Fraktion in dem thermischen Schritt des ersten katalytisch-thermischen Umsetzungsverfahrens und die wenigstens eine zweite Fraktion in den thermischen Schritt des zweiten katalytisch-thermischen Umsetzungsverfahrens zurückzuführen.

Vorteilhafterweise ist eine entsprechende Anlage zur Durchführung eines Verfahrens eingerichtet, wie es zuvor erläutert wurde, und weit hierzu entsprechende Mittel auf. Auf die obigen Erläuterungen bzgl. Merkmalen und Vorteilen des erfindungsgemäßen Verfahrens sei daher ausdrücklich verwiesen.

Die Erfindung wird nachfolgend unter Bezugnahme auf die beigefügte Zeichnung erläutert, welche eine bevorzugte Ausführungsform der Erfindung veranschaulicht.

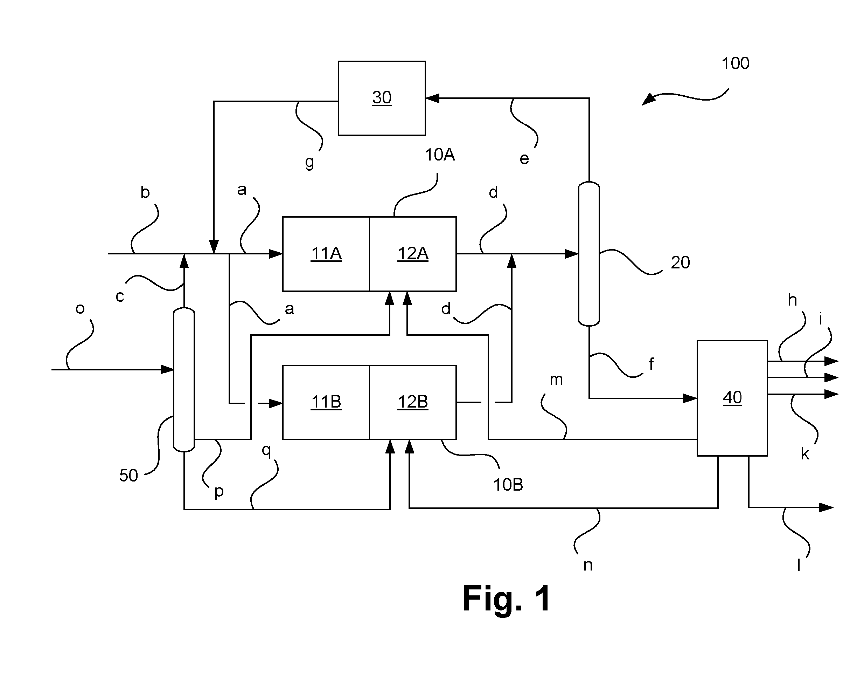

Kurze Beschreibung der Zeichnung
- In Figur 1: ist ein Prozess gemäß einer Ausführungsform der Erfindung schematisch veranschaulicht.

### Ausführliche Beschreibung der Zeichnung

In Figur 1 ist ein Prozess gemäß einer Ausführungsform der Erfindung schematisch veranschaulicht und insgesamt mit 100 bezeichnet. Zentraler Bestandteil des Prozesses 100 sind ein erstes katalytisch-thermisches Umsetzungsverfahren 10A und ein zweites katalytisch-thermisches Umsetzungsverfahren 10B, denen jeweils ein Methan und Sauerstoff enthaltender Einsatzstrom a zugeführt wird. Die Einsatzströme a können dabei aus einem gemeinsamen Ausgangsstrom bereitgestellt werden, der beispielsweise unter Verwendung eines sauerstoffreichen Stroms b und eines methanreichen Stroms c sowie unter Verwendung eines weiteren methanreichen Stroms g gebildet werden kann.

Das erste katalytisch-thermische Umsetzungsverfahren 10A und das zweite katalytisch-thermische Umsetzungsverfahren 10B werden parallel durchgeführt, d. h. es gibt im Wesentlichen keinen stofflichen Austausch zwischen dem ersten und dem zweiten katalytisch-thermischen Umsetzungsverfahren 10A bzw. 10B. Die katalytischthermischen Umsetzungsverfahren 10A bzw. 10B umfassen jeweils einen katalytischen Schritt 11A bzw. 11 B und einen sich dem katalytischen Schritt anschließenden thermischen Schritt 12A bzw. 12B. Das Methan, das über die Einsatzströme a jeweils in die katalytischen Schritte 11A bzw. 11 B eingespeist wird, wird dort jeweils zu einem Teil unter Bildung von höheren Kohlenwasserstoffen umgesetzt, wodurch jeweils ein erstes Gasgemisch gebildet wird, das die erwähnten höheren Kohlenwasserstoffe umfasst, und das direkt jeweils dem sich anschließendem thermischen Schritt 12A bzw. 12B zugeführt wird.

In den thermischen Schritten 12A bzw. 12B werden die höheren Kohlenwasserstoffe in den ersten Gasgemischen jeweils zu einem Teil weiter umgesetzt, wodurch jeweils ein zweites Gasgemisch gebildet wird, das Ethan, Ethylen und Paraffine und Olefine mit drei und vier Kohlenstoffatomen umfasst. Ein derartiges zweites Gasgemisch kann jeweils in Form eines Stroms d aus dem thermisch-katalytischen Umsetzungsverfahren 10A bzw. 10B abgezogen und vor der Einspeisung in ein Trennverfahren 20 zu einem Sammelstrom vereinigt werden, der hier nicht gesondert bezeichnet ist. In dem Trennverfahren 20 kann dabei aus den zweiten Gasgemischen, die neben den genannten Kohlenwasserstoffen auch Kohlendioxid und Kohlenmonoxid enthalten, ein kohlendioxid- und/oder kohlenmonoxidreicher Strom e gebildet werden, der einem Methanisierungsverfahren 30 zugeführt werden kann. Ein verbleibendes, kohlendioxid- und kohlenmonoxidarmes Kohlenwasserstoffgemisch f kann einem weiteren Trennverfahren 40 zugeführt werden. In dem Methanisierungsverfahren 30 wird aus dem Kohlenmonoxid und/oder Kohlendioxid Methan gebildet, das in Form eines methanreichen Stroms g den Einsatzströmen a zugespeist und damit in die thermischkatalytischen Umsetzungsverfahren 10A bzw. 10B zurückgeführt werden kann.

Der kohlendioxid- und kohlenmonoxidarme Kohlenwasserstoffstrom f wird in der weiteren Trenneinrichtung 40 weiter aufgetrennt, wobei insbesondere Produktfraktionen gebildet und in Form der Ströme h, i und k abgezogen werden können. Es handle sich beispielsweise um einen ethylenreichen Strom h, einen propylenreichen Strom i und/oder einen butadienreichen Strom k. In dem weiteren Trennverfahren 40 wird ferner eine Fraktion gebildet, die Kohlenwasserstoffe mit fünf oder fünf und mehr Kohlenstoffatomen umfasst, und die beispielsweise in Form des Stroms I abgezogen werden kann.

Ein wesentlicher Aspekt der vorliegenden Erfindung ist die Verwendung unterschiedlicher Spaltbedingungen in dem thermischen Schritt 12A des ersten thermisch-katalytischen Umsetzungsverfahrens 10A und dem thermischen Schritt 12B des zweiten thermisch-katalytischen Umsetzungsverfahrens 10B. Der thermische Schritt 12A des ersten thermisch-katalytischen Umsetzungsverfahrens 12 wird dabei bei einer höheren Spaltschärfe, der thermische Schritt 12B des zweiten katalytischthermischen Umsetzungsverfahrens bei einer niedrigeren Spaltschärfe durchgeführt. Dies lässt die individuelle Behandlung von Komponenten aus dem weiteren Trennverfahren 40 zu. So kann beispielsweise in dem weiteren Trennverfahren 40 auch eine Fraktion gebildet werden, die überwiegend oder ausschließlich Ethan enthält. Diese kann als Strom m abgezogen und in den thermischen Schritt 12A des ersten thermisch-katalytischen Umsetzungsverfahren 10A geführt werden, wo es mit höherer Spaltschärfe gespalten werden kann. Entsprechend kann in dem weiteren Trennverfahren 40 auch eine Fraktion gebildet werden, die Paraffine mit drei und/oder vier Kohlenstoffatomen und/oder Olefine mit vier Kohlenstoffatomen enthält. Diese kann in Form des Stroms n aus dem weiteren Trennverfahren 40 abgezogen und in den thermischen Schritt 12B des zweiten thermisch-katalytischen Umsetzungsverfahrens 10B geführt werden.

Der methanreiche Strom c wird in dem erfindungsgemäßen Prozess 100 unter Verwendung eines Abtrennverfahrens 50 aus einem Erdgasstrom o hergestellt. In einem entsprechenden Abtrennprozess können neben dem methanreichen Strom c, wie hier in Form der Ströme p und q veranschaulicht, weitere Fraktionen in dem Abtrennverfahren 50 gebildet werden. Beispielsweise können diese Ströme p und q Kohlenwasserstoffe mit unterschiedlicher Kohlenstoffzahl umfassen, die gezielt in einen der thermischen Schritte 12A bzw. 12B geführt werden können. Selbstverständlich können höhere Kohlenwasserstoffe aus Erdgas auch aus dem Verfahren ausgeleitet werden.

## Patentansprüche

1. Prozess (100) zur Herstellung von Olefinen, bei dem Methan zusammen mit Sauerstoff parallel einem ersten und einem zweiten katalytisch-thermischen Umsetzungsverfahren (10A, 10B) zugeführt wird, wobei das erste und das zweite katalytisch-thermische Umsetzungsverfahren (10A, 10B) jeweils einen katalytischen Schritt (11A, 11 B) und einen sich dem katalytischen Schritt anschließenden thermischen Schritt (12A, 12B) umfassen, wobei
- das Methan in den katalytischen Schritten (11A, 11 B) jeweils zu einem Teil unter Bildung von höheren Kohlenwasserstoffen umgesetzt wird, wodurch jeweils ein erstes Gasgemisch gebildet wird, das die höheren Kohlenwasserstoffe umfasst,
- die ersten Gasgemische jeweils in den thermischen Schritten (12A, 12B) vollständig oder teilweise einem absteigenden Temperaturgradienten unterworfen werden, wodurch die höheren Kohlenwasserstoffe jeweils zu einem Teil weiter umgesetzt werden und jeweils ein zweites Gasgemisch gebildet wird, das Ethan, Ethylen und Paraffine und Olefine mit drei und vier Kohlenstoffatomen umfasst,
- aus den zweiten Gasgemischen eine erste Fraktion, die überwiegend oder ausschließlich Ethan enthält, und wenigstens eine zweite Fraktion, die Paraffine mit drei und/oder vier Kohlenstoffatomen und/oder Olefine mit vier Kohlenstoffatomen enthält, gebildet werden,
- in dem thermischen Schritt (12A) des ersten katalytisch-thermischen Umsetzungsverfahrens (10A) eine höhere Spaltschärfe festgelegt wird als in dem thermischen Schritt (12B) des zweiten katalytisch-thermischen Umsetzungsverfahrens (10B), und
- die erste Fraktion in den thermischen Schritt (12A) des ersten katalytischthermischen Umsetzungsverfahrens (10A) und die wenigstens eine zweite Fraktion in den thermischen Schritt (12B) des zweiten katalytisch-thermischen Umsetzungsverfahrens (10B) zurückgeführt wird.

2. Prozess (100) nach Anspruch 1, bei dem die Spaltschärfe in dem thermischen Schritt (12A) des ersten katalytisch-thermischen Umsetzungsverfahrens (10A) derart festgelegt wird, dass Ethan mindestens zu 20 und höchstens zu 60 Molprozent umgesetzt wird.

3. Prozess (100) nach Anspruch 1 oder 2, bei dem die Spaltschärfe in dem thermischen Schritt (12B) des zweiten katalytisch-thermischen Umsetzungsverfahrens (10B) derart festgelegt wird, dass Propan mindestens zu 30 und höchstens zu 80 Molprozent umgesetzt wird.

4. Prozess (100) nach einem der vorstehenden Ansprüche, bei dem das in dem katalytischen Schritt (11A) des ersten katalytisch-thermischen Umsetzungsverfahrens (10A) gebildete erste Gasgemisch dem thermischen Schritt (12A) des ersten katalytisch-thermischen Umsetzungsverfahrens (10A) auf einem Temperaturniveau von 880 bis 950°C zugeführt wird.

5. Prozess (100) nach einem der vorstehenden Ansprüche, bei dem das in dem katalytischen Schritt (11 B) des zweiten katalytisch-thermischen Umsetzungsverfahrens (10B) gebildete erste Gasgemisch dem thermischen Schritt (12B) des zweiten katalytisch-thermischen Umsetzungsverfahrens (10B) auf einem Temperaturniveau von 850 bis 950 °C zugeführt wird.

6. Prozess (100) nach einem der vorstehenden Ansprüche, bei dem die zweite Fraktion Propan, Butan und Butene, insbesondere lineare Butene, umfasst.

7. Prozess (100) nach einem der vorstehenden Ansprüche, bei die zweiten Gasgemische Kohlenmonoxid und/oder Kohlendioxid enthalten, und bei dem das Kohlenmonoxid und/oder Kohlendioxid aus dem zweiten Gasgemisch abgetrennt, einer Methanisierung (40) unterworfen, und zumindest einem der katalytischthermischen Umsetzungsverfahren (10A, 10B) wieder zugeführt wird.

8. Prozess (100) nach einem der vorstehenden Ansprüche, bei dem die zweiten Gasgemische Isobuten enthalten, und bei dem das Isobuten zu einem tert-Butylether umgesetzt wird.

9. Prozess (100) nach einem der vorstehenden Ansprüche, bei dem aus den zweiten Gasgemischen eine ethylenreiche, eine propylenreiche und/oder eine butadienreiche Fraktion abgetrennt werden.

10. Prozess (100) nach einem der vorstehenden Ansprüche, bei dem die zweiten Gasgemische Kohlenwasserstoffe mit fünf oder fünf und mehr Kohlenstoffatomen enthalten, und bei dem aus den zweiten Gasgemischen eine an solchen Kohlenwasserstoffen reiche Fraktion abgetrennt wird.

11. Prozess (100) nach einem der vorstehenden Ansprüche, bei dem das den katalytisch-thermischen Umsetzungsverfahren (10A, 10B) zugeführte Methan zumindest zum Teil in einem Abtrennverfahren (50) aus Erdgas abgetrennt wird.

12. Prozess (100) nach Anspruch 11, bei dem in dem Abtrennverfahren (50) eine Fraktion gebildet wird, die Kohlenwasserstoffe mit zwei oder zwei und mehr Kohlenstoffatomen enthält, und bei dem diese Fraktion teilweise oder vollständig dem thermischen Schritt (12A, 12B) zumindest eines der katalytisch-thermischen Umsetzungsverfahren (10A, 10B) zugeführt wird.

13. Anlage zur Durchführung eines Prozesses (100) zur Herstellung von Olefinen, die dafür eingerichtet ist, Methan zusammen mit Sauerstoff parallel einem ersten und einem zweiten katalytisch-thermischen Umsetzungsverfahren (10A, 10B) zuzuführen, wobei das erste und das zweite katalytisch-thermische Umsetzungsverfahren (10A, 10B) jeweils einen katalytischen Schritt (11A, 11 B) und einen sich dem katalytischen Schritt anschließenden thermischen Schritt (12A, 12B) umfassen, wobei die Anlage dafür eingerichtet ist,
- das Methan in den katalytischen Schritten (11A, 11 B) jeweils zu einem Teil unter Bildung von höheren Kohlenwasserstoffen umzusetzen, wodurch jeweils ein erstes Gasgemisch gebildet wird, das die höheren Kohlenwasserstoffe umfasst,
- die ersten Gasgemische jeweils in den thermischen Schritten (12A, 12B) vollständig oder teilweise einem absteigenden Temperaturgradienten zu unterwerfen, wodurch die höheren Kohlenwasserstoffe jeweils zu einem Teil weiter umgesetzt werden und jeweils ein zweites Gasgemisch gebildet wird, das Ethan, Ethylen und Paraffine und Olefine mit drei und vier Kohlenstoffatomen umfasst,
- aus den zweiten Gasgemischen eine erste Fraktion, die überwiegend oder ausschließlich Ethan enthält, und wenigstens eine zweite Fraktion, die Paraffine mit drei und/oder vier Kohlenstoffatomen und/oder Olefine mit vier Kohlenstoffatomen enthält, zu bilden,
- in dem thermischen Schritt (12A) des ersten katalytisch-thermischen Umsetzungsverfahrens (10A) eine höhere Spaltschärfe festzulegen als in dem thermischen Schritt (12B) des zweiten katalytisch-thermischen Umsetzungsverfahrens (10B), und
- die erste Fraktion in den thermischen Schritt (12A) des ersten katalytischthermischen Umsetzungsverfahrens (10A) und die wenigstens eine zweite Fraktion in den thermischen Schritt (12B) des zweiten katalytisch-thermischen Umsetzungsverfahrens (10B) zurückzuführen.

14. Anlage nach Anspruch 13, die zur Durchführung eines Verfahrens nach einem der Ansprüche 1 bis 12 eingerichtet ist und hierzu entsprechende Mittel aufweist.
